# EUROPÄISCHE PATENTSCHRIFT

(11) **EP 0 394 793 B1**
(45) Veröffentlichungstag und Bekanntmachung des Hinweises auf die Patenterteilung: **15.06.1994**
(21) Anmeldenummer: 90107143.1
(22) Anmeldetag: 13.04.1990
(51) Int. Cl.: C07C 61/29, C07C 51/44, C07C 51/48, C07C 51/41, C08F 2/26

(54) **Abietinsäurederivate und ihre Verwendung als Elmulgatoren**
Abietic acid derivatives and their utilization as emulsifying agents
Dérivés de l'acide abiétique et leur utilisation comme émulsifiants

(30) Priorität: 26.04.1989 DE 3913680; 08.08.1989 DE 3926118
(43) Veröffentlichungstag der Anmeldung: 31.10.1990
(73) Patentinhaber: BAYER AG, 51368 Leverkusen (DE)
(72) Erfinder: Piejko, Karl-Erwin, Dr., D-5060 Bergisch-Gladbach 2 (DE); Constant, Dieter, Dr., D-5090 Leverkusen 3 (DE); Lindner, Christian, Dr., D-5000 Koeln 91 (DE); Wulff, Claus, Dr., D--4150 Krefeld (DE)

(56) Entgegenhaltungen:
- US-A- 2 296 503
- US-A- 4 450 260
- METHODEN DER ORGANISCHEN CHEMIE, Band XIV, Teil 1, 1961, Georg Thieme Verlag, Stuttgart, DE; HOUBEN-WEYL, "Makromolekulare Stoffe, S. 194-196

## Beschreibung

Die Erfindung betrifft ein Verfahren zur Vorbehandlung von Abietinsäurederivaten und die Verwendung der erfindungsgemäß behandelten Abietinsäurederivate als Emulgatoren für die Emulsionspolymerisation olefinischer Monomerer.

Abietinsäurederivate (Harzseifen) weisen gute Emulgiereigenschaften auf und beeinflussen mit ihrer Hilfe hergestellte Polymerisate dadurch günstig, daß im Polymerisat verbleibende Restmengen die Verarbeitungseigenschaften wie Klebrigkeit und Spritzgießverhalten verbessern.

Abietinsäurederivate (Harzseifen) werden aus Holzharzen wie Kolophonium gewonnen, die als Hauptbestandteile Terpenverbindungen vom Typ der Abietinsäure enthalten. Um brauchbare Emulgatoren zu erhalten, werden diese Harze üblicherweise einer Hydrierung oder Dehydrierung unterworfen (siehe Houben-Weyl "Methoden der organischen Chemie" Bd. XIV, 4. Auflage, S. 194f, Georg Thieme Verlag, Stuttgart 1961). Die so erhaltenen modifizierten Harzsäuren bzw. deren Salze (Abietinsäurederivate) werden großtechnisch als Emulgatoren, z.B. bei der Herstellung von Kaltkautschuk, eingesetzt. Sie sind kommerziell erhätlich unter Handelsnamen wie Dresinate®, Gresinox®.

Die Abietinsäurederivate können auch zur Herstellung von ABS-Pfropfpolymerisaten, insbesondere zur Herstellung der Kautschukgrundlage für diese Pfropfprodukte und als Hilfsemulgatoren für die eigentliche Pfropfpolymerisation eingesetzt werden. ABS-Pfropfpolymerisate werden in größtem Umfang hergestellt und dienen als solche oder mit anderen thermoplastischen Kunststoffen (Polyvinylchlorid, Polycarbonat) gemischt als thermoplastische Formmassen.

Aus mit Abietinsäurederivaten als Emulgatoren hergestellten Polymerisaten dampfen bei Hitzebeanspruchung Begleitstoffe aus. Diese Erscheinung wird allgemein als "Fogging" bezeichnet, weil die aus dem Kunststoff des Armaturenbretts ausgedampften Stoffe als Belag auf der Windschutzscheibe von Kraftfahrzeugen beobachtet wurden.

Die als Emulgatoren benutzten Abietinsäurederivate sind komplexe Produktgemische, weil sie durch chemische Abwandlung eines komplex zusammengesetzten Naturproduktes entstehen. Auch Reinigungsoperationen, die erst zu den bekannten, kommerziell erhältlichen Produkten mit brauchbaren Emulgatoreigenschaften führen, verhindern das "Fogging" nicht.

Gegenstand der Erfindung ist ein Verfahren zur Vorbehandlung von Abietinsäurederivaten (Harzseifen), das dadurch gekennzeichnet ist, daß man sie unter Zugabe von Basen in Wasser löst und eine Extraktion oder Wasserdampfdestillation durchführt.

Die Lösung der Abietinsäurederivate kann bei 20 bis 95°C mit einer Konzentration von 1 Gew.-% bis zur Sättigung, bevorzugt von 2 bis 25 Gew.-%, unter Zusatz von Basen wie Natronlauge oder Kalilauge hergestellt werden, Der pH der Lösung ist bevorzugt 10 bis 14, insbesondere 11 bis 13.

Die Lösung der Abietinsäurederivate kann mit organischen Medien, die nicht voll wassermischbar sind, bei einer Temperatur von 20°C bis zum Siedepunkt des organischen Mediums in bekannter Weise ausgeschüttelt oder kontinuierlich extrahiert werden. Die organischen Medien dürfen mit der Abietinsäurederivatlösung keine stabilen Emulsionen ausbilden, weil sich sonst die organische Phase nicht mehr abtrennen läßt. Beispiele für organische Medien sind Alkane und Alkangemische, bevorzugt in Kombination mit kleinen Mengen höherer Alkohole, wie Hexan, Cyclohexan, Heptan, Petrolethergemische mit 1 bis 10 Gew.-% Butanolen, Pentanolen oder Hexanolen, Ether wie Dibutylether, Diethylether, aliphatische Ester wie Butylacetat, Propylacetat, Ethylacetat, sowie Mischungen dieser Flüssigkeiten.

Besonders bevorzugte organische Medien sind Hexan, Cyclohexan und Heptan mit 1 bis 10 Gew.-% Butanolen.

Es ist möglich, bekannte Hilfsmittel zum Abtrennen der organischen Phase einzusetzen, z.B. Silikonöle.

Bei der Extraktion müssen die oben angegebenen pH-Werte der wäßrigen Phase erhalten bleiben. Besonders wenn organische Phasen verwendet werden, die unter Verbrauch von Alkali verseifen können (z.B. Ester), müssen niedrige Temperaturen eingehalten und gegebenenfalls der pH-Wert durch Zugabe von Lauge korrigiert werden.

Bei der Behandlung der Abietinsäurederivatlösung durch Wasserdampfdestillation verfährt man in üblicher Weise bevorzugt, indem man überhitzten Dampf einleitet unter Beibehaltung der Ausgangskonzentration an Abietinsäurederivat. In einer bevorzugten Ausführungsform der Wasserdampfdestillation werden bekannte phenolische Inhibitoren, beispielsweise sterisch gehinderte Phenole, insbesondere solche mit Molmassen über 200 g/Mol, in Mengen von 10 bis 1.000 ppm zugesetzt. Die Menge des Destillats ist bevorzugt das einfache bis dreißigfache der Menge der eingesetzten Lösung.

Die Extraktion und die Wasserdampfdestillation müssen erfindungsgemäß so durchgeführt werden, daß der Extrakt nach Entfernen des organischen Mediums bzw das Wasserdampfdestillat 0,5 bis 25 Gew.-%, bevorzugt 3 bis 20 Gew.-%, insbesondere 5 bis 15 Gew.-%, des ursprünglich eingesetzten Abietinsäurederivats enthält

Nach der erfindungsgemäßen Behandlung der Abietinsäurederivatlösung kann die Löslichkeit der Abietinsäurederivate in der alkalischen Lösung verringert sein. Dies steht der Verwendung als Emulgator jedoch nicht entgegen, da die volle Löslichkeit wiederhergestellt werden kann durch Erwärmen, Verdünnen oder Zugabe eines Lösungsvermittlers. Als Lösungsvermittler sind z.B. die oben für die Extraktion genannten organischen Medien geeignet. In bevorzugten Fällen, beispielsweise mit Alkanen, erhält man nach der Extraktion eine wäßrige Phase, die bereits geringe Mengen der Alkane enthält. Diese Menge ist häufig ausreichend, um eine klare Lösung zu bilden.

Es ist auch möglich, den Emulgator aus der behandelten Lösung zu isolieren, z.B. durch Erniedrigung des pH oder durch Abdampfen des Wassers. Zur weiteren Verwendung bei der Emulsionspolymerisation kann dann der Emulgator in Monomeren gelöst und so eingebracht werden. Man muß dann die für eine gute Emulgatorwirkung notwendige Menge an Base separat als wäßrige Lösung zugeben. Bevorzugte Vorbehandlung ist die Extraktion.

Da die erfindungsgemäß eingesetzten Abietinsäurederivate in den zur Extraktion verwendeten Lösungsmitteln voll löslich sein können, ist es überraschend, daß bei der erfindungsgemäßen Extraktion eine selektive Entfernung bestimmter Anteile möglich ist. Offenbar werden überwiegend die Bestandteile herausgenommen, die das "Fogging" verursachen.

Ein weiterer Gegenstand der Erfindung ist die Verwendung der erfindungsgemäß vorbehandelten Abietinsäurederivate als Emulgatoren für die Emulsionspolymerisation olefinischer Monomerer.

Beispiele von an sich bekannten Polymerisaten, die mit den erfindungsgemäßen Emulgatoren hergestellt werden können, sind vernetzte und unvernetzte Dien- und Acrylatkautschuke, z.B. Dienkautschuke aus Butadien, Isopren, Chloropren, gegebenenfalls mit Comonomeren wie Styrol und/oder Acrylnitril und Acrylatkautschuke aus Alkylacrylaten geebenenfalls mit Comonomeren wie Acrylnitril, Styrol, Methylmethacrylat und/oder Pfropfpolymerisate von harzbildenden Monomeren auf Dien- und auf Acrylatkautschuke, z.B. Acrylnitril/Butadien/Styrol-, Acrylnitril/Styrol/Alkylacrylat- oder Methylmethacrylat/Butadien/Styrol-Polymerisate und/oder harzartige Polymerisate von Styrol, α-Methylstyrol, C₁-C₄-Alkylmethacrylat, C₁-C₄-Vinylcarbonsäuren, Acrylnitril, C₁-C₈-Alkylacrylaten und Mischungen daraus.

Die Polymerisationsverfahren unter Einsatz der erfindungsgemäßen Emulgatoren sind bekannt, ebenso die Aufarbeitung der Emulsionspolymerisate zu Polymerisatpulvern und Granulaten.

Bei der Herstellung der Polymerisate können die erfindungsgemäßen Emulgatorlösungen z.T. durch übliche anionische Emulgatoren wie Alkylcarbonsäuren, Alkylsulfonsäuren, bekannte Abietinsäurederivate etc. substituiert sein in Anteilen bis zu 60 % bezogen auf die eingesetzten Abietinsäurederivate, bevorzugt jedoch in Anteilen kleiner 40 %.

Bei mehrstufigen Polymerisationsverfahren wie zur Herstellung von Pfropfpolymerisaten sind bei Mitverwendung nicht erfindungsgemäßer Emulgatoren solche Verfahren bevorzugt, bei denen der Anteil erfindungsgemäßer Emulgatoren besonders in den letzten Verfahrensschritten größer 70 %, insbesondere größer 90 % ist.

Die erfindungsgemäßen Emulsionspolymerisate werden nach üblichen Methoden zu Pulvern aufgearbeitet, z.B. durch Koagulation, Sprühtrocknung oder Ausdampfverfahren. Die resultierenden Polymerisatpulver weisen als solche oder in Kombination mit anderen Thermoplasten verbesserte Verarbeitungs- und Gebrauchseigenschaften auf, da während der Verarbeitung in der Schmelze oder aus dem fertigen Formteil weniger flüchtige Anteile herausdampfen und somit geringere Maschinenbeläge, eine geringere Geruchsbelästigung oder beispielsweise bei Anwendungen im Kraftfahrzeug weniger Beläge an den Innenseiten der Scheiben auftreten, also das "Fogging" vermieden wird.

### Beispiele

1. Herstellung der erfindungsgemäßen Abietinsäurederivatlösungen
   Als Abietinsäurederivate wurden die unter dem Handelsnamen Dresinate® 731 und Gresinox® 578M erhältlichen Produkte (Natriumsalz, 70 %ig in Wasser) eingesetzt.
1.1 Extraktion mit Hexan/i-Butanol
   A Eine Lösung aus 240 g Dresinate® 731, 20 g Natriumhydroxid (fest) in 2.400 g Wasser wird mit einer Mischung aus 710 g Hexan und 43 g i-Butanol ausgeschüttelt. Die wäßrige Phase (Lösung 1.1.A) besitzt einen pH von 12,6. Die organische Phase enthält 12,5 g Extrakt.
   B 1.650 g einer analog A behandelten wäßrigen Phase werden noch zweimal mit je 650 g einer Mischung aus Hexan mit 4 % i-Butanol ausgeschüttelt. Die resultierende wäßrige Phase (Lösung 1.1.B) besitzt einen pH von 12,6. Die vereinigten organischen Phasen enthalten 1,55 g Extrakt.

Die Tatsache, daß bei dem zweiten Extraktionsprozeß mit erhöhten Mengen an organischer Phase nur noch geringe Extraktmengen erhalten werden, zeigt, daß beim erfindungsgemäßen Verfahren nur bestimmte Anteile des Emulgatorgemisches extrahiert werden, die Extraktion demnach selektiv ist.
1.2 Extraktion mit Essigester
   Eine Lösung aus 240 g Dresinate® 731, 20 g Natriumhydroxid (fest) in 2.400 g Wasser wird mit 1,1 l Essigester ausgeschüttelt. Die wäßrige Phase wird anschließend am Rotationsverdampfer bei 70°C andestilliert (Destillatmenge 400 ml) Der pH der resultierenden wäßrigen Phase beträgt nur noch 8,8. Zur weiteren Verwendung als Emulgatorlösung werden 8,6 g 1n Natronlauge pro 100 g Lösung zugesetzt. Die resultierende wäßrige Lösung (Lösung 1.2) besitzt einen pH von 12. Die organische Phase enthält 22,7 g Extrakt.
1.3 Wasserdampfdestillation
   240 g Dresinate® 731, 20 g Natriumhydroxid (fest) und 480 mg 2,2′-Methylen-bis-(4-methyl-6-cyclohexylphenol) werden in 700 g Wasser bei 70°C gelöst und einer Wasserdampfdestillation unterworfen, so daß das Volumen der Sumpfvorlage ungefähr konstant bleibt. Es wird so lange destilliert, bis ca, 6,5 l Wasser als Destillat übergegangen sind. Der Rückstand wird mit Wasser zu einer 10 %igen Lösung an Dresinate® verdünnt (Lösung 1.3). Der pH der Lösung beträgt 12,6. Das Destillat wird mit Essigester extrahiert; die Menge an festem Extrakt beträgt 7,2 g.
2. Herstellung von erfindungsgemäßen und Vergleichs-Pfropfpolymerisaten vom ABS-Typ
   In einem Reaktor werden vorgelegt: 896 g Wasser, 303 g eines Polybutadienkautschuklatex mit einem Feststoffgehalt von 49,5 Gew.-% und einem mittleren Teilchendurchmesser (d₅₀) von 390 nm und 355 g eines Polybutadienkautschuklatex mit einem Feststoffgehalt von 42,3 Gew.-% und einem mittleren Teilchendurchmesser (d₅₀) von 130 nm. (Diese Sl-Polybutadiene wurden mit Dresinate® als Emulgator hergestellt.) Es wird 15 Min. mit Stickstoff gespült. Nach Aufheizen auf 65°C wird unter leichtem Stickstoffstrom eine Lösung aus 7,9 g Kaliumperoxodisulfat in 286 g Wasser zugegeben. Danach werden folgende Lösungen gleichmäßig innerhalb von 7 Stunden in den Reaktor eingespeist:
   - Lösung 1:: 462 g Styrol
   238 g Acrylnitril
   - Lösung 2:: Emulgatorlösung, Zusammensetzung siehe Tabelle 1.

   Man läßt 6 Stunden bei 65°C auspolymerisieren, fügt 1 Gew.-% (bezogen auf Feststoff) 2,2′-Methylen-bis-(4-methyl-6-cyclohexylphenol) zu und koaguliert den Latex mit einer wäßrigen Magnesiumsulfatlösung. Das Koagulat wird mit Wasser gewaschen und bei 60°C bis zu einer Restfeuchte < 0,5 % getrocknet.
3. Prüfung der Eigenschaften
   Die gemäß 2. erhaltenen Pulver werden einer Foggingprüfung (Windowfoggingtest; 3 Stunden bei 100°C) nach DIN 75201 unterworfen. Die Resultate sind ebenfalls in Tabelle 1 aufgeführt.

**Tabelle 1**

| Zusammensetzung der Emulgatorlösung (Lösung 2) zur Herstellung der ABS-Pfropfpolymerisate und Glanzwerte der entsprechenden Pulver aus Foggingmessungen | | |
|---|---|---|
| Beispiel Nr. | Lösung 2 Typ/Menge | Glanzwerte in % |
| 1 | Beispiel 1.1.A/367 g | 91 |
| 2 | Beispiel 1.1.B/382 g | 93 |
| 3 | Beispiel 1.2 /251 g + 70 g Wasser | 91 |
| 4 | Beispiel 1.3 /227 g + 100 g Wasser | 88 |
| 5* | Dresinate 731® 31,3 g + 220 g Wasser + 74 g 1n NaOH | 78 |
| 6* | Gresinox 578M® 21,9 g + 230 g Wasser + 74 g 1n NaOH | 85 |

| | | |
|---|---|---|
| * = Vergleichsversuche | | |

Die Glanzwerte in Tabelle 1 zeigen, daß mit den erfindungsgemäßen Emulgatorlösungen Emulsionspolymerisate vom ABS-Typ mit verminderten flüchtigen Anteilen hergestellt werden können. Daraus hergestellte Formteile zeichnen sich ebenfalls durch verminderte flüchtige Anteile aus.

## Patentansprüche

1. Verfahren zur Vorbehandlung von Abietinsäurederivaten (Harzseifen), dadurch gekennzeichnet, daß man sie unter Zugabe von Basen in Wasser löst und eine Extraktion oder Wasserdampfdestillation durchführt.

2. Verwendung der vorbehandelten Abietinsäurederivate gemäß Anspruch 1 als Emulgatoren für die Emulsionspolymerisation olefinischer Monomerer.

## Claims

1. A process for the pretreatment of abietic acid derivatives (resin soaps), characterized in that they are dissolved in water with addition of bases and then subjected to extraction or stripping with steam.

2. The use of the pretreated abietic acid derivatives according to claim 1 as emulsifiers for the emulsion polymerization of olefinic monomers.

## Revendications

1. Procédé de prétraitement de dérivés d'acide abiétique (savons de résine), caractérisé en ce qu'on dissout ces dérivés dans l'eau avec addition de bases et on conduit une extraction ou une distillation à la vapeur d'eau.

2. Utilisation des dérivés d'acide abiétique prétraités suivant la revendication 1 comme émulsifiants pour la polymérisation en émulsion de monomères oléfiniques.
